(19) Europäisches Patentamt
European Patent Office
Office européen des brevets

(11) EP 3 120 851 A1

(12) **EUROPEAN PATENT APPLICATION**

(43) Date of publication:
**25.01.2017 Bulletin 2017/04**

(21) Application number: **15382375.2**

(22) Date of filing: **21.07.2015**

(51) Int Cl.:
***A61K 31/519*** *(2006.01)* *A61P 35/00* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **Pangaea Biotech S.L.**
  **08028 Barcelona (ES)**
- **Institut Químic de Sarriá CETS Fundació Privada**
  **08017 Barcelona (ES)**

(72) Inventors:
- **MOLINA VILA, Miguel Ángel**
  **E-08028 Barcelona (ES)**
- **GARCÍA ROMÁN, Silvia**
  **E-08028 Barcelona (ES)**
- **BORRELL BILBAO, José Ignacio**
  **E-08017 Barcelona (ES)**
- **TEIXIDO CLOSA, Jordi**
  **E-08017 Barcelona (ES)**
- **ESTRADA TEJEDOR, Roger**
  **E-08017 Barcelona (ES)**
- **PUIG DE LA BELLACASA CAZORLA, Raimon**
  **E-08017 Barcelona (ES)**

(74) Representative: **ABG Patentes, S.L.**
**Avenida de Burgos, 16D**
**Edificio Euromor**
**28036 Madrid (ES)**

(54)
# 4-AMINO-6-(2,6-DICHLOROPHENYL)-8-METHYL-2-(PHENYLAMINO)-PYRIDO[2,3-D]PYRIMIDIN-7(8H)-ONE FOR TREATMENT OF SOLID CANCERS

(57) The present invention relates to 4-amino-6-(2,6-dichlorophenyl)-8-methyl-2-(phenylamino)-pyrido[2,3-*d*]pyrimidin-7(8*H*)-one of formula (1) for use in the treatment and/or prevention of solid tumors, and combinations comprising said compound and a chemotherapeutic drug.

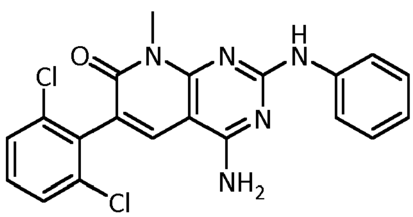

(1)



Printed by Jouve, 75001 PARIS (FR)

## Description

### FIELD OF THE INVENTION

The present invention relates to 4-amino-6-(2,6-dichlorophenyl)-8-methyl-2-(phenylamino)-pyrido[2,3-*d*]pyrimidin-7(8*H*)-one for use in the treatment and/or prevention of solid cancers, to combinations comprising said compound and a chemotherapeutic drug, to their use in medicine, in particular for the treatment and/or prevention of solid tumors, and to pharmaceutical compositions comprising said compound.

### BACKGROUND OF THE INVENTION

During the last decade, cancer has become one of the most frequent diseases with more than 14.1 million of new cases per year worldwide, according to 2012 WHO (World Health Organization) report [GLOBALCAN2012, WHO, Globalcan2012: Estimated cancer incidence, mortality and prevalence worldwide in 2012]. According to WHO, about 30% of cancers could be detected at an early stage due to improved diagnostic methodologies enhancing the probabilities of a successful therapeutic treatment.

Cancers are caused by malignant tumors formed by an abnormal growth of cells and tissue leading to organ failure. They fall into two main categories: solid cancers and hematological cancers.

Solid cancers or solid tumors are neoplasms (new growth of cells) or lesions (damage of anatomic structures or disturbance of physiological functions) formed by an abnormal growth of body tissue cells other than blood, bone marrow or lymphatic cells. A solid tumor consists of an abnormal mass of cells which may stem from different tissue types such as liver, colon, breast, or lung, and which initially grows in the organ of its cellular origin. However, such cancers may spread to other organs through metastatic tumor growth in advanced stages of the disease.

In contrast, hematological cancers or tumors are cancer types affecting blood, bone marrow, and lymph nodes. Hematological tumors may derive from either of the two major blood cell lineages: myeloid and lymphoid cell lines. The myeloid cell line normally produces granulocytes, erythrocytes, thrombocytes, macrophages, and mast cells, whereas the lymphoid cell line produces B, T, NK and plasma cells. Lymphomas (e.g. non-Hodgkin's Lymphoma), lymphocytic leukemias, and myeloma are derived from the lymphoid line, while acute and chronic myelogenous leukemia (AML, CML), myelodysplastic syndromes and myeloproliferative diseases are myeloid in origin.

Main treatment options for solid cancers include surgery, chemotherapy, radiation therapy, hormonal therapy, targeted therapy and palliative care. The efficacy of chemotherapy depends on the type of cancer and the stage. In combination with surgery, chemotherapy has proven useful in a number of different solid cancer types including: breast cancer, colorectal cancer, pancreatic cancer, osteogenic sarcoma, testicular cancer, ovarian cancer, and certain lung cancers. The effectiveness of chemotherapy is often limited by toxicity to other tissues in the body. Even when it is impossible for chemotherapy to provide a permanent cure, chemotherapy may be useful to reduce symptoms like pain or to reduce the size of an inoperable tumor in the hope that surgery will be possible in the future.

Lung cancer is a solid malignant tumor characterized by uncontrolled cell growth in tissues of the lung. Lung cancer is the leading cause of cancer-related death worldwide. WHO defines lung cancer as tumors arising from the respiratory epithelium and divides them into four major cell types; Small-cell lung carcinoma (SCLC), adenocarcinoma, squamous cell carcinoma, and large-cell carcinoma. Historically, the major pathological distinction was simply between SCLC and the others which were grouped and referred to as non-small cell lung carcinoma (NSCLC). SCLC tumors grow faster, spread earlier and are more sensitive to cytotoxic chemotherapeutic agents. All histologic types of lung cancer can be found in current and former smokers although squamous and small-cell carcinomas are most commonly associated with heavy tobacco use. Squamous carcinoma (SCC) was the most commonly diagnosed subtype of NSCLC through the first half of the 20th century. However, the decline in cigarette consumption and changes in their composition made adenocarcinoma the most frequent histologic subtype of lung cancer. In lifetime never-smokers, women, and younger adults (< 60 years), adenocarcinoma tends to be the dominant histology.

In advanced non-small cell lung carcinoma (NSCLC), chemotherapy improves survival and is used as first-line treatment, provided the person is well enough for the treatment. Typically, two drugs are used, of which one is often platinum-based (either cisplatin or carboplatin). Other commonly used drugs are gemcitabine, paclitaxel, docetaxel, pemetrexed, etoposide or vinorelbine.

Until recently, there was no need to distinguish among the various subtypes of NSCLC as there were no clear differences in therapeutic outcome based on histology alone. This perspective radically changed in 2004 with the discovery of the epidermal growth factor receptor (EGFR) mutation which is present in lung adenocarcinoma and is related to a favorable response to EGFR tyrosine kinase inhibitors (TKI). Anaplastic lymphoma kinase (ALK) fusions were later found to also be limited to adenocarcinoma and current guidelines recommend initial EGFR and ALK testing in patients with adenocarcinoma or mixed tumors with adenocarcinoma components.

In 2004, three separate investigative teams demonstrated an association between the presence of EGFR

mutations (deletions in exon 19 or point mutations in exon 21) that activate the EGFR by altering the ATP binding site and response to gefitinib. These mutations were found to be more common in females, in light or never smokers in patients of Asian ethnicity and were nearly always in adenocarcinoma histology. Subsequently, randomized trials demonstrated that EGFR TKI produced higher response rates, less toxicity, and improved progreesion free survival (PFS) rates compared with platinum-based doublets for chemotherapy-naive patients with advanced lung cancer having these mutations. [R. Rosell et al., Lancet Oncol, 2012, 13:239-246]. Unfortunately, TKI-treated patients with EGFR mutations are not cured by the EGFR TKI therapy, and progress after a median of 9 to 10 months.

EML4-ALK was the first targetable fusion oncokinase to be identified in 4-6% of lung adenocarcinomas. EML4-ALK generates a transforming tyrosine kinase which represents a novel molecular target in a small subset of NSCLCs. Patients with EML4-ALK positive tumors are characteristically younger age, female, and never to light smokers [E.L. Kwak et al., N Engl J Med, 2010, 363:1693-1703; and T. Takahashi et al., Ann Surg Oncol, 2010, 17:889-897]. Based on data from a phase I clinical trial which showed an overall response rate of 57% and a probability of progression-free survival at 6 months of 72%, crizotinib has been approved by the U.S. Food and Drug Administration for the treatment of NSCLC patients with ALK transforming rearrangements [E.L. Kwak et al., NEngl J Med, 2010, 363:1693-1703].

Several ALK inhibitors have demonstrated in vitro dual inhibitory activity against c-ros oncogene 1 kinase (ROS1), an orphan tyrosine kinase receptor protooncogene that forms fusions and which defines another clinically actionable oncogenic driver mutation in NSCLC [K. Rikova et al., Cell, 2007, 131:1190-1203; and S.H. Ou et al., Expert Rev Anticancer Ther, 2012, 12:447-456]. It has been recently reported that approximately 1.4% of NSCLCs harbor ROS1 rearrangements [K. Takeuchi et al., Nat Med, 2012, 18:378-381]. Patients with ROS1 rearrangements are also significantly younger, more likely to be never-smokers and are more often diagnosed with the histological subtype of adenocarcinoma with wide distribution of tumor grade [K. Bergethon et al., J Clin Oncol, 2012, 30:863-870]. Recently, a report from investigators at the Massachusetts General Hospital Cancer Center has showed that ROS 1-driven tumors can be treated with crizotinib.

Despite available treatments, there remains a need for new clinically effective agents for the treatment of solid cancers, in particular for the treatment of carcinomas, such as lung cancer.

4-Amino-2-arylamino-6-(2,6-dichlorophenyl)-pyrido[2,3-d]pyrimidin-7(8*H*)-ones have been disclosed as being active against non-Hodgkin's lymphomas, i.e. hematological tumors [R. Puig de la Bellacasa et al., Eur. J. Med. Chem., 2014, 86, 664-675]. Among the synthesized compounds disclosed in said paper, 4-amino-6-(2,6-dichlorophenyl)-8-methyl-2-(phenylamino)-pyrido[2,3-*d*]pyrimidin-7(8*H*)-one of formula (1) was selected for further development due to its good cytostatic activity in mantle cell lymphoma (MCL) cell lines, which belong to the group of non-Hodgkin's lymphomas, i.e. hematological tumors [R. Puig de la Bellacasa et al., Eur. J. Med. Chem., 2014, 86, 664-675].

## SUMMARY OF THE INVENTION

The inventors have surprisingly found that a compound of formula (1)

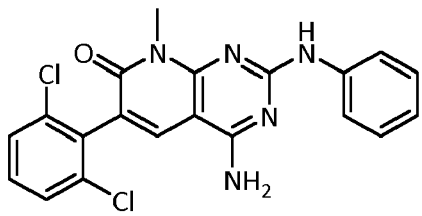

(1)

i.e., 4-amino-6-(2,6-dichlorophenyl)-8-methyl-2-(phenylamino)-pyrido[2,3-*d*]pyrimidin-7(8*H*)-one, has anticancer activity in several solid tumor cell lines such as lung cancer, colorectal cancer, pancreatic cancer, larynx cancer, tongue cancer and prostate cancer, as shown in the examples (Table 2).

Thus, in the first aspect the present invention relates to a compound of formula (1) (i.e. 4-amino-6-(2,6-dichlorophenyl)-8-methyl-2-(phenylamino)-pyrido[2,3-*d*]pyrimidin-7(8*H*)-one) or a pharmaceutically acceptable salt or solvate thereof for use in the treatment and/or prevention of solid cancers.

In a second aspect, the present invention relates to a combination comprising a compound of formula (1) or a pharmaceutically acceptable salt or solvate thereof and a chemotherapeutic drug.

In a third aspect, the present invention relates to a combination comprising a compound of formula (1) or a pharmaceutically acceptable salt or solvate thereof and a chemotherapeutic drug for use in medicine.

In a fourth aspect, the present invention relates to a combination as defined in the second aspect for use as defined in the first aspect, i.e. a combination comprising a compound of formula (1) or a pharmaceutically acceptable

salt or solvate thereof and a chemotherapeutic drug for use in the treatment and/or prevention of solid cancers.

In a fifth aspect, the present invention relates to a composition comprising a compound of formula (1) or a pharmaceutically acceptable salt or solvate thereof, a chemotherapeutic drug and a pharmaceutically acceptable excipient.

In another aspect, the invention relates to a compound of formula (1) (i.e. 4-amino-6-(2,6-dichlorophenyl)-8-methyl-2-(phenylamino)-pyrido[2,3-*d*]pyrimidin-7(8*H*)-one) or a pharmaceutically acceptable salt or solvate thereof for use in the preparation of a medicament, for the prevention and/or treatment solid cancers.

In another, the invention relates to a method for treating and/or preventing a solid cancer in a subject in need of such treatment, comprising administering and effective amount of a compound of formula (1) (i.e. 4-amino-6-(2,6-dichlorophenyl)-8-methyl-2-(phenylamino)-pyrido[2,3-*d*]pyrimidin-7(8*H*)-one) or a pharmaceutically acceptable salt or solvate thereof.

In another aspect, the invention relates to a combination comprising a compound of formula (1) or a pharmaceutically acceptable salt or solvate thereof and a chemotherapeutic drug for use in the preparation of a medicament, in particular for the prevention and/or treatment solid cancers.

In another aspect, the invention relates to a method for treating and/or preventing a solid cancer in a subject in need of such treatment, comprising administering an effective amount of a compound of formula (1) or a pharmaceutically acceptable salt or solvate thereof, and an effective amount of a chemotherapeutic drug. In one aspect, the compound of formula (1), or a pharmaceutically acceptable salt or solvate thereof, and the chemotherapeutic agent are administered in a synergistically effective amount.

## DESCRIPTION OF THE INVENTION

In a first aspect, the present invention relates to a compound of formula (1) (i.e. 4-amino-6-(2,6-dichlorophenyl)-8-methyl-2-(phenylamino)-pyrido[2,3-*d*]pyrimidin-7(8*H*)-one) or a pharmaceutically acceptable salt or solvate thereof for use in the treatment and/or prevention of solid cancers.

Alternatively, the invention relates to a compound of formula (1) (i.e. 4-amino-6-(2,6-dichlorophenyl)-8-methyl-2-(phenylamino)-pyrido[2,3-*d*]pyrimidin-7(8*H*)-one) or a pharmaceutically acceptable salt or solvate thereof for use in the preparation of a medicament, for the prevention and/or treatment solid cancers.

Alternatively, the invention relates to a method for treating and/or preventing a solid cancer in a subject in need of such treatment, comprising administering and effective amount of a compound of formula (1) (i.e. 4-amino-6-(2,6-dichlorophenyl)-8-methyl-2-(phenylamino)-pyrido[2,3-*d*]pyrimidin-7(8*H*)-one) or a pharmaceutically acceptable salt or solvate thereof.

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric, sulfuric, phosphoric, diphosphoric, hydrobromic, hydroiodic, and nitric acid, and organic acids, for example, citric, fumaric, maleic, malic, mandelic, ascorbic, oxalic, succinic, tartaric, benzoic, acetic, methanesulfonic (mesylate), ethanesulfonic, benzenesulfonic (besylate), or p-toluenesulfonic (tosylate) acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines. For instance, pharmaceutically acceptable salts of compounds provided herein are synthesized from the parent compound which contains a basic or an acid moiety by conventional chemical methods. Generally, such salts are, for example, prepared by reacting the free base or free acid forms of these compounds with a stoichiometric amount of the appropriate acid or base, respectively, in water or in an organic solvent or in a mixture of the two.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a $^{13}C$- or $^{14}C$-enriched carbon or $^{15}N$-enriched nitrogen are within the scope of this invention.

Unless otherwise stated, the compounds of the invention are also meant to include tautomers which differ only in the position of a proton and a single bond adjacent to a double bond (as shown below), such as amine-imine tautomers, enamine-imine, lactam-lactim tautomer.

As used herein, the term "solvate" means any form of the compound according to the invention which has another molecule (most likely a polar solvent) attached to it via non-covalent bonding. Examples of solvates include hydrates and alcoholates, e.g. methanolate.

The compound of formula (1) can be obtained following the procedure described by Puig de la Bellacasa et al. [Eur. J. Med. Chem., 2014, 86, 664-675], which is incorporated herein by reference with respect to the synthesis of said compound of formula (1) i.e. 4-amino-6-(2,6-dichlorophenyl)-8-methyl-2-(phenylamino)-pyrido[2,3-*d*]pyrimidin-7(8*H*)-one.

As used herein, the terms "treat", "treatment" and "treating" refer to the reduction or amelioration of the progression, severity and/or duration of a disease or disorder, such as a solid tumor, or the amelioration of one or more symptoms (preferably, one or more discernible symptoms) related a disease or disorder, such as a solid tumor, e.g. reduction or stabilization of tumor size or cancerous cell count.

As used herein, the terms "prevent", "prevention" and "preventing" refer to the reduction in the risk of acquiring or developing a given disease or disorder, e.g., a solid tumor, or the reduction or inhibition of the recurrence or a disease or disorder, e.g., a solid tumor.

As used herein, the terms "subject", "patient" are used interchangeably. The terms "subject" and "patient" refer to an animal (e.g., a bird such as a chicken, quail or turkey, or a mammal), preferably a mammal including a non-primate (e.g., a cow, pig, horse, sheep, rabbit, guinea pig, rat, cat, dog, and mouse) and a primate (e.g., a monkey, chimpanzee and a human), and more preferably a human. In one embodiment, the subject is a non-human animal such as a farm animal (e.g., a horse, cow, pig or sheep), or a pet (e.g., a dog, cat, guinea pig or rabbit). In another embodiment, the subject is a human.

The term "cancer" or "tumor", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. Cancers usually share some of the following characteristics: sustaining proliferative signaling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumor cells resemble, which is therefore presumed to be the origin of the tumor.

As used herein, the term "solid cancer" or "solid tumor" refers to an abnormal growth of body tissue cells other than blood, bone marrow or lymphatic cells. Examples of solid tumors are carcinomas, sarcomas, germinomas and blastomas.

Carcinomas are cancers derived from epithelial cells and account for 80% to 90% of all cancer cases since epithelial tissues are most abundantly found in the body. This group includes many of the most common cancers, particularly in the aged, and include lung cancer, colorectal cancer, pancreatic cancer, larynx cancer, tongue cancer, prostate cancer, breast cancer, ovarian cancer, liver cancer, head and neck cancer, esophageal cancer, renal cancer, endometrial cancer, gall bladder cancer, bladder cancer and gastric cancer. Carcinomas are of two types: adenocarcinoma and squamous cell carcinoma. Adenocarcinoma develops in an organ or gland and squamous cell carcinoma originates in squamous epithelium. Adenocarcinomas may affect mucus membranes and are first seen as a thickened plaque-like white mucosa. These are rapidly spreading cancers.

Sarcomas are cancers arising from connective tissue including muscles, bones, cartilage and fat. Examples of sarcomas include osteosarcoma (of the bone), chondrosarcoma (of the cartilage), leiomyosarcoma (smooth muscles), rhabdomyosarcoma (skeletal muscles), mesothelial sarcoma or mesothelioma (membranous lining of body cavities), fibrosarcoma (fibrous tissue), angiosarcoma or hemangioendothelioma (blood vessels), liposarcoma (adipose or fatty tissue), glioma or astrocytoma (neurogenic connective tissue found in the brain), myxosarcoma (primitive embryonic connective tissue) and mesenchymous or mixed mesodermal tumor (mixed connective tissue types).

Germinomas refer to germ cell tumors, derived from pluripotent cells, most often presenting in the testicle or the ovary (seminoma and dysgerminoma, respectively).

Blastomas are cancers derived from immature precursor cells or embryonic tissue. Blastomas are more common in children than in older adults. Examples of blastomas include hepatoblastoma, medulloblastoma, nephroblastoma, pancreatoblastoma, pleruropulmonary blastoma, retinoblastoma and glioblastoma multiforme.

Cancers that can be treated or prevented by the methods of the present invention are solid tumors, e.g. lung cancer, colorectal cancer, pancreatic cancer, larynx cancer, tongue cancer, breast cancer, ovarian cancer, prostate cancer, liver cancer, head and neck cancer, esophageal cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, dysgerminoma, embryonal carcinoma, Wilms' tumor, cervical cancer, testicular tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma,

retinoblastoma.

Preferably, the solid cancer is selected form the group consisting of carcinoma, glioblastoma multiforme, astrocytoma, oligodendroglioma and ependymoma. More preferably, the solid cancer is a carcinoma. More preferably, the solid tumor is selected from the group consisting of a lung cancer, colorectal cancer, pancreatic cancer, larynx cancer, tongue cancer, prostate cancer, breast cancer, ovarian cancer, liver cancer, head and neck cancer, esophageal cancer, renal cancer, endometrial cancer, gall bladder cancer, bladder cancer gastric cancer and skin cancer. Still more preferably, the solid tumor is selected from the group consisting of lung cancer colorectal cancer, pancreatic cancer prostate cancer, head and neck cancer and skin cancer.

Preferably, the solid cancer is lung cancer. The term "lung cancer", refers to a cancer that originates in the lungs. As explained above, according to histological characteristics lung cancer can be classified in two main groups: non-small cell lung cancer (NSCLC) and small cell lung cancer (SCLC). In the context of the present invention, "lung cancer" or "lung tumor" is understood as any type of tumor damage of the lung tissue, including NSCLC and SCLC.

Malignant non-small cell epithelial tumors of the lung are classified by the World Health Organization (WHO)/International Association for the Study of Lung Cancer (IASLC). There are three main subtypes of non-small cell lung cancer (NSCLC): squamous cell carcinoma, adenocarcinoma and large cell carcinoma.

Squamous cell carcinoma of the lung refers to a carcinoma with observable features and characteristics indicative of squamous differentiation. Most squamous cell carcinomas of the lung are located centrally, in the larger bronchi of the lung. Squamous cell carcinomas are linked more strongly with smoking than other forms of NSCLC.

Adenocarcinomas of the lung refer to carcinomas featuring microscopic glandular-related tissue cytology, tissue architecture and/or gland-related molecular products, and have been classified by the WHO/IASLC as: well-differentiated fetal adenocarcinoma, mucinous (colloid) adenocarcinoma, mucinous cystadenocarcinoma, signet ring adenocarcinoma and clear cell adenocarcinoma.

Large cell carcinomas of the lung are composed of large, monotonous rounded or overtly polygonal-shaped cells with abundant cytoplasm. They also include several uncommon variants such as large-cell neuroendocrine carcinoma (LCNEC), basaloid carcinoma, lymphoepithelioma-like carcinoma, clear cell carcinoma and large cell carcinoma with rhabdoid phenotype.

Classification of subtypes of SCLC include: small cell carcinoma and combined small cell carcinoma (i.e., SCLC combined with neoplastic squamous and/or glandular components).

Preferably, the lung cancer is non-small cell lung cancer (NSCLC). Alternatively, the lung cancer is small cell lung cancer (SCLC).

In a particular embodiment, the NSCLC is selected from squamous cell lung carcinoma, large cell lung carcinoma and lung adenocarcinoma, preferably squamous cell carcinoma.

In a particular embodiment, the solid cancer, preferably a carcinoma, more preferably lung cancer, even more preferably NSCLC, still more preferably wherein the NSCLC is lung adenocarcinoma, large cell carcinoma, or squamous cell lung carcinoma, more preferably wherein the NSCLC is selected from the group consisting of a solid cancer having ALK translocations, ROS-1 translocations, KRAS mutations, RET translocations, DDR2 mutations, EGFR mutations, FGFR1 amplification, SOX2 amplification and/or PIK3CA mutations, and a solid cancer having over expression of DDR2, EGFR and/or EPHA3.

In a particular embodiment, the solid cancer, preferably a carcinoma, more preferably lung cancer, even more preferably NSCLC, still more preferably wherein the NSCLC is lung adenocarcinoma, large cell carcinoma, or squamous cell lung carcinoma, more preferably wherein the NSCLC is selected from the group consisting of a cancer having ALK translocations (such as EML4-ALK fusions, in particular E13;A20 (v1), E20;A20 (V2), E6a/b;A20 (v3a/b), E14;A20 (v4), E2a/b;A20 (v5a/b), E13b;A20 (v6), E14;A20 (v7), E15;A20 ("v4"), and E18;A20 ("v5"); KIF5B-ALK fusions; and TFG-ALK fusions), ROS-1 translocations (such as SLC34A2-ROS1, CD74-ROS1, EZR-ROS1, TPM3-ROS1, and SDC4-ROS1) and/or KRAS mutations (such as KRAS c.34G>T (p.G12C), KRAS c.34G>C (p.G12R), KRAS c.34G>A (p.G12S), KRAS c.35G>C (p.G12A), KRAS c.35G>A (p.G12D), KRAS c.35G>T (p.G12V), KRAS c.37G>T (p.G13C), KRAS c.37G>C (p.G13R), KRAS c.37G>A (p.G13S), KRAS c.38G>C (p.G13A), KRAS c.38G>A (p.G13D), KRAS c.181C>A (p.Q61K), KRAS c.182A>T (p.Q61L), KRAS c.182A>G (p.Q61R), KRAS c.183A>C (p.Q61H), and KRAS c.183A>T (p.Q61H)), BRAF mutations (such as c.1799T>A (p.V600E) and c.1798_1799GT>AA (p.V600K)), and RET translocations (like KIF5B-RET, CCDC6-RET, NCOA4-RET, and TRIM33-RET).

In addition, the solid cancer, preferably a carcinoma, more preferably lung cancer, even more preferably NSCLC, still more preferably wherein the NSCLC is adenocarcinoma or squamous cell carcinoma, is selected from the group consisting of a cancer having EGFR mutations in exons 18, 19, 20, 21 mainly insertions, deletions and codon mutations such as G719S (c.2155G>A), G719C (c.2155G>T), G719A (c.2155G>C), L858R (c.2573T>G), and L861Q (c.2582T>A).

In addition, the solid cancer, preferably a carcinoma, more preferably lung cancer, even more preferably NSCLC, still more preferably wherein the NSCLC is squamous cell carcinoma, is selected from the group consisting of a cancer having DDR2 mutations (such as c.716T>G (p.L239R), c.1912A>T (p.I638F) and c.1960C>U (p.T654M)), FGFR1 amplification, SOX2 amplification or PIK3CA mutations (such as c.1624G>A (p.E542K), c.1633G>A (p.E545K), c.3140A>G

(p.H1047R) and c.3140A>T (p.H047L)).

Translocations may be readily identified in tumor tissue by reverse transcription-polymerase chain reaction or fluorescent in situ hybridization, amplifications by fluorescent in situ hybridization and mutations by a variety of techniques such as polymerase chain reaction followed by Sanger sequencing or allelic discrimination with specific probes (Molina-Vila et al.. J Thorac Oncol. 2008 ;3(11):1224-35).

The term "mutation", as used herein refers to is a permanent change of the nucleotide sequence of the genome of an organism, virus, or extrachromosomal DNA or other genetic elements. Mutations result from damage to DNA which is not repaired or to RNA genomes (typically caused by radiation or chemical mutagens), errors in the process of replication, or from the insertion or deletion of segments of DNA by mobile genetic elements. Mutations may or may not produce discernible changes in the observable characteristics (phenotype) of an organism. Mutations play a part in both normal and abnormal biological processes including: evolution, cancer, and the development of the immune system, including junctional diversity. Mutation can result in several different types of change in sequences. Mutations in genes can either have no effect, alter the product of a gene, or prevent the gene from functioning properly or completely. Mutations can also occur in nongenic regions.

The term "translocation", as used herein refers to is a chromosome abnormality caused by rearrangement of parts between nonhomologous chromosomes. A gene fusion may be created when the translocation joins two otherwise-separated genes. It is detected on cytogenetics or a karyotype of affected cells. Translocations can be balanced (in an even exchange of material with no genetic information extra or missing, and ideally full functionality) or unbalanced (where the exchange of chromosome material is unequal resulting in extra or missing genes).

The term "amplification", as used herein refers to any duplication of a region of DNA that contains a gene. Gene duplications can arise as products of several types of errors in DNA replication and repair machinery as well as through fortuitous capture by selfish genetic elements. Common sources of gene duplications include ectopic homologous recombination, retrotransposition event, aneuploidy, polyploidy, and replication slippage

In addition, the solid cancer, preferably a carcinoma, more preferably lung cancer, even more preferably NSCLC, still more preferably wherein the NSCLC is squamous cell carcinoma, is selected from the group consisting of a cancer having over expression of DDR2, EGFR, and EPHA3. Overexpression of DDR2, EGFR and EPHA3 can be identified by immunohistochemistry (IHQ). Immunohistochemistry or IHC refers to the process of detecting antigens (e.g., proteins) in cells of a tissue section by exploiting the principle of antibodies binding specifically to antigens in biological tissues. Specific molecular markers are characteristic of particular cellular events such as proliferation or cell death (apoptosis). Visualising an antibody-antigen interaction can be accomplished in a number of ways. In the most common instance, an antibody is conjugated to an enzyme, such as peroxidase, that can catalyse a colour-producing reaction (such as in immunoperoxidase staining). Alternatively, the antibody can also be tagged to a fluorophore, such as fluorescein or rhodamine (such as in immunofluorescence) (Ferri et al., Am J Pathol. 2004;164(5):1575-85).

Furthermore, the compound of formula (1) or a pharmaceutically acceptable salt or solvate thereof for use according to the present invention is applicable to a subject who suffers from any of the above mentioned solid cancers at stages 0, I, II, III or IV. Stage 0 is used to describe cancer *in situ*; these cancers are still located in the place they started and have not invaded nearby tissues. Stage I is a small cancer or tumor that has not grown deeply into nearby tissues and has not spread to the lymph nodes or other parts of the body. Stage II and III indicate cancers or tumors that are larger in size, have grown more deeply into nearby tissue, and have spread to lymph nodes, but not to other parts of the body. Stage IV means that the cancer has spread to other organs or parts of the body. It may also be called advanced or metastatic cancer.

The term "metastasis" or "metastatic", as used herein, refers to the spread of a cancer from one organ or tissue to another non-adjacent organ or tissue. Cancer cells can break away, leak, or spill from a primary tumor and enter lymphatic and blood vessels, circulate throughout the bloodstream, and be deposited elsewhere in the body. This occurrence is referred to as "metastasis". Metastasis is one of the hallmarks of malignancy of cancers.

As used herein, the term "effective amount" refers to an amount of a compound of this invention which is sufficient to reduce or ameliorate the severity, duration, progression, or onset of a the solid cancers described above, prevent the advancement of a the solid cancers described above, cause the regression of the solid cancers described above, prevent the recurrence, development, onset or progression of a symptom associated with the solid cancers described above, or enhance or improve the prophylactic or therapeutic effect(s) of another therapy. The precise amount of compound administered to a subject will depend on the mode of administration, the type and severity of the solid cancer and on the characteristics of the subject, such as general health, age, sex, body weight and tolerance to drugs. It will also depend on the degree, severity and type of cell proliferation, and the mode of administration. The skilled artisan will be able to determine appropriate dosages depending on these and other factors. When co-administered with other agents, e.g., when co-administered with another chemotherapeutic agent, an "effective amount" of the second agent (chemotherapeutic agent) will depend on the type of drug used. Suitable dosages are known for approved agents and can be adjusted by the skilled artisan according to the condition of the subject, the type of condition(s) being treated and the amount of a compound of the invention being used. In cases where no amount is expressly noted, an effective

amount should be assumed.

Non-limiting examples of an effective amount of the compound of formula (1) or a pharmaceutically acceptable salt or solvate thereof are provided herein below, expressed as the equivalent amount of compound of formula (1) in free form, i.e. not forming a salt or a solvate. In a specific embodiment, the invention provides a method of treating and/or preventing solid tumors or one or more symptoms thereof, said method comprising administering to a subject in need thereof a dose (expressed as amount of compound per kg of body weight) of at least about 150 $\mu$g/kg, preferably at least about 250 $\mu$g/kg, at least about 500 $\mu$g/kg, at least about 1 mg/kg, at least about 5 mg/kg, at least about 10 mg/kg, at least about 25 mg/kg, at least about 50 mg/kg, at least about 75 mg/kg, at least about 100 mg/kg, at least about 125 mg/kg, at least about 150 mg/kg, or at least about 200 mg/kg or more of a compounds of formula (1) or a pharmaceutically acceptable salt or solvate thereof. The compounds of formula (1) or a pharmaceutically acceptable salt or solvate thereof once every day, preferably, once every 2 days, once every 3 days, once every 4 days, once every 5 days, once every 6 days, once every 7 days, once every 8 days, once every 10 days, once every two weeks, once every three weeks, or once a month.

The compound of formula (1) or a pharmaceutically acceptable salt or solvate thereof may be administered in a pharmaceutical composition comprising said compound and a pharmaceutically acceptable excipient.

By "pharmaceutically acceptable" such as in the recitation of a "pharmaceutically acceptable excipient" or a "pharmaceutically acceptable salt" is meant herein a material that is not biologically or otherwise undesirable, i.e. the material may be incorporated into a pharmaceutical composition administered to a patient without causing any undesirable biological effects or interacting in a deleterious manner with any of the other components of the composition in which it is contained.

The term "excipient" refers to a diluent, adjuvant, carrier, or vehicle with which the active ingredient is administered and includes any such materials known in the art that are nontoxic and do not interact with other components of a pharmaceutical composition or drug delivery system in a deleterious manner. Such pharmaceutical excipients can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

For its administration to a subject, such as a mammal, e.g., a human, in need of treatment, the pharmaceutical composition of the invention may be administered by any appropriate route, such as, oral (e.g., oral, sublingual, etc.), parenteral (e.g., subcutaneous, intramuscular, intravenous, etc.), respiratory (e.g., inhalation or insufflation), rectal, or local administration (e.g. implants, transdermal, etc.).

Examples of pharmaceutical compositions include any solid (tablets, pills, capsules, granules etc.) or liquid (solutions, suspensions or emulsions) composition for oral or parenteral administration. The mentioned formulations will be prepared using standard methods such as those described or referred to in the European and US Pharmacopoeias and similar reference texts.

The pharmaceutical compositions may be in oral form, either solid or liquid. Suitable dose forms for oral administration may be tablets, capsules, syrups or solutions and may contain conventional excipients known in the art such as binding agents, for example syrup, acacia, gelatine, sorbitol, tragacanth, or polyvinylpyrrolidone; fillers, for example lactose, sugar, maize starch, calcium phosphate, sorbitol or glycine; tableting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate. The solid oral compositions may be prepared by conventional methods of blending, filling or tablet pharmaceutical production. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The compounds and compositions of this invention may be used with other drugs to provide a combination therapy, in particular in combination with another chemotherapeutic agent. The other drugs may form part of the same composition, or be provided as a separate composition for administration at the same time or at different time. In this sense, whenever it is said that a composition comprises a certain drug combination, it is meant that the composition comprises the drugs contained in the drug combination, i.e. the drugs of the drug combination form part of the composition.

Thus, in another aspect, the present invention relates to a combination comprising a compound of formula (1) or a pharmaceutically acceptable salt or solvate thereof and a chemotherapeutic drug.

As used herein, the terms "combination" or "combination product" are used interchangeably and refer to a product comprising (a) the compound of formula (1) or a pharmaceutically acceptable salt or solvate thereof and (b) the chemotherapeutic drug either in a single composition or forming part of different compositions.

In another aspect, the invention relates a combination comprising a compound of formula (1) or a pharmaceutically acceptable salt or solvate thereof and a chemotherapeutic drug for use in medicine.

In a further aspect the present invention relates to a combination comprising a compound of formula (1) or a pharmaceutically acceptable salt or solvate thereof and a chemotherapeutic drug for use as defined in the first aspect of the present invention.

Illustrative, non- limitative examples of chemotherapeutic drugs are crizotinib (3-[(1R)-1-(2,6-dichloro-3-fluorophenyl)ethoxy]-5-(1-piperidin-4-ylpyrazol-4-yl)pyridin-2-amine), ceritinib (5-Chloro-$N^2$-[2-isopropoxy-5-methyl-4-(4-piperidinyl)phenyl]-$N^4$-[2-(isopropylsulfonyl)phenyl]-2,4-pyrimidinediamine) alectinib (9-ethyl-6,6-dimethyl-8-(4-morpholinopiperidin-1-yl)-11-oxo-6,11-dihydro-5H-benzo[b]carbazole-3-carbonitrile), erlotinib (TARCEVA), gefitinib (IRESSA), ABX-EGF, lapatinib, canertinib, ZD-6474 (ZACTIMA), AEE788, sorafenib, foretinib ($N^{1'}$-[3-fluoro-4-[[6-methoxy-7-(3-morpholinopropoxy)-4-quinolyl]oxy]phenyl]-N'-(4-fluorophenyl)cyclopropane-1,1-dicarboxamide), Genistein (4',5,7-trihydroxyisoflavone), KY12420, bevacizumab (AVASTIN), sunitinib (SUTENT), vatalanib, anecortave (RETAANE), trastuzumab (HERCEPTIN), alemtuzumab (CAMPATH), gemtuzumab (MYLOTARG, hP67.6, anti-CD33,), rituximab (RITUXAN), tositumomab (BEXXAR, anti-CD20), oregovomab (OVAREX), ibritumomab tiuxetan (ZEVALIN), cetuximab (ERBITUX), epratuzumab (LYMPHOCIDE), olaparib, etoposide, topotecan, teniposide, mitoxantrone, cisplatin, mechlorethamine, cyclophosphamide, ifosfamide, melphalan, chorambucil, busulfan, thiotepa, carmustine, lomustine, carboplatin, dacarbazine, procarbazine, bleomycin, doxorubicin, daunorubicin, idarubicin, mitomycin, vincristine, vinblastine, cytarabine, methotrexate, hydroxyurea, 5-fluorouracil, floxuridine, 6-thioguanine, 6-mercaptopurine, fludarabine, pentostatin, and chlorodeoxyadenosine, and lenalidomide

Preferred chemotherapeutic drugs for combining with the compound of formula (1) or a pharmaceutically acceptable salt or solvate thereof are EGFR TKIs (gefitinib, erlotinib, icotinib hydrochloride, foretinib, rociletinib, ABT-414, ASP8273, EGF-816, PF-06747775, AZD3759, HM61713, LY-3164530, NRC2694, canertinib, and TAS 120), ALK TKIs (crizotinib, ceritinib, alectinib, entrectinib, gliteritinib fumarte, PF-06463922, CEP-37440, EBI-215, AZD3463, CEP28122, CM-118" TSR-011, X-396, DLX-521, AP26113), anti-EGFR monoclonal antibodies (e.g cetuximab, panitumumab, nimotuzumab, necitumumab, ABT-806, duligotuzumab, futuximab, imgatuzumab, and GC-1118A), anti-VEGF monoclonal antibodies (e.g bevacizumab, ramucirumab, icrucumab, tanibirumab, vanucizumab, LY3022856, MP0250, OMP305B83, sevacizumab, and VGX100), fusion proteins (aflibercept), multi-tyrosine kinase inhibitors (e.g afatinib, imatinib, dacomitinib, dasatinib, ponatinib, KD-019, bosutinib, lapatinib ditosylate, AZD9291, neratinib, poziotinib, S-222611, suramin hexasodium, AL-6802, BGB-102, PB357, Pyrotinib, sunitinib, sorafenib tosylate, pazopanib, regorafenib, apatinib, axitinib, carbozantinib, lenvatinib, nintedanib, vandetanib, tivozanib, anlotinib, midostaurin, muparfostat, BMS-690514, ENMD-2076, golvatinib, lucitanib, motesanib, necuparinib, RAF265, famitinib, telatinib, X82, ALNVSP, altiratinib, ABT348, MGCD516, OB318, ODM203, HHGV678, LY-3012207, CS2164, ilorasertib, radotinib, bafetinib, NRCAN-019, ABL001, metatinib tromethamine, rebastinib tosylate, VX-15, Herceptin), Immuno inhibitors (alemtuzumab, gemtuzumab, rituximab, tositumomab, zevalin, epratuzumab, ipilumumab), others (oregovomab, olaparib, etoposide, topotecan, teniposide, mitoxantrone, cisplatin, carboplatin, mechlorethamine, cyclosphosphamide, ifosfamide, melphalan, chlorambucil, busulfan, thiotepa, carmustine, lomustine, decarbazine, procarbazine, bleomycin, doxorubicin, daunorubicin, idarubicin, mitomycin, vincristine, vinblastine, cytarabine, hydroxyurea, 5-fluorouracil, 6-thioguanine, irinotecan, fludarabine, pentostatin, cladribine, lenalidomide, paclitaxel, abraxane, and docetaxel). Preferably, the chemotherapeutic drug for combining with the compound of formula (1) or a pharmaceutically acceptable salt or solvate thereof is crizotinib.

A synergistic effect has been found when the compound of formula (1) is used in combination with crizotinib for the treatment of lung cancer, in particular NSCLC, more particularly in the treatment of lung adenocarcinoma.

The compound of formula (1) or a pharmaceutically acceptable salt or solvate thereof and the chemotherapeutic drug, as defined above, may be administered simultaneously, sequentially or separately. In any of these particular administration modes, the combination according to the present invention is designed to enable the simultaneous, sequential or separate administration compound of formula (1) or a pharmaceutically acceptable salt or solvate thereof and the chemotherapeutic drug. In a particular embodiment, the compound of formula (1) or a pharmaceutically acceptable salt or solvate thereof and the chemotherapeutic drug, as defined above, are administered simultaneously. In another particular embodiment, the compound of formula (1) or a pharmaceutically acceptable salt or solvate thereof and the chemotherapeutic drug, as defined above, are administered sequentially. In another particular embodiment, the compound of formula (1) or a pharmaceutically acceptable salt or solvate thereof and the chemotherapeutic drug, as defined above, are administered separately.

Simultaneous administration may, e.g. take place in the form of one composition comprising the compound of formula (1) or a pharmaceutically acceptable salt or solvate thereof and the chemotherapeutic drug, as defined above, or by simultaneously administering, i.e. administering at the same time, the compound of formula (1) or a pharmaceutically acceptable salt or solvate thereof and the chemotherapeutic drug, as defined above, which are formulated independently, i.e. not forming part of the same composition.

Sequential administration means administration of the compound of formula (1) or a pharmaceutically acceptable salt or solvate thereof and the chemotherapeutic drug, as defined above, at a different time point, in a chronically

staggered manner.

Separate administration means administration of the compound of formula (1) or a pharmaceutically acceptable salt or solvate thereof and the chemotherapeutic drug, as defined above, independently of each other at different time points.

When administered sequentially or separately, the compound of formula (1) or a pharmaceutically acceptable salt or solvate thereof and the chemotherapeutic drug, as defined above, may be in any order. In one particular embodiment, the compound of formula (1) or a pharmaceutically acceptable salt or solvate thereof is administered first. In another particular embodiment, the chemotherapeutic drug, as defined above, is administered first.

A further aspect of the invention relates to a combination comprising a compound of formula (1) or pharmaceutically acceptable salt or solvate thereof and a chemotherapeutic drug, as defined above, and a pharmaceutically acceptable excipient (as defined above), preferably in the form of a pharmaceutical composition comprising these components.

The following examples represent specific embodiments of the present invention. They do not intend to limit in any way the scope of the invention defined in the present description.

## Examples

### Example 1. Kinase inhibition profile

In a first experiment, the kinase inhibition profile of the compound of formula (1) was evaluated at Proqinase (http://www.proqinase.com) by measuring residual activity values at a concentration of 10 μM of the test compound in singlicate in front of the following kinases: ABL2, EPHA3, CSF1-R, DDR2 wt, DDR2 N456 S, EGF-R d746-750, EGF-R d747-752/P753S, EGF-R G719C, EGF-R G719S, EGF-R L858R, EGF-R L861Q, EGF-R wt, EPHA1, EPHA2, EPHB1, ERBB4, LCK, MINK1, RAF1 Y340D/Y341D (untagged), RIPK2, STK17A. In a second experiment, the kinase inhibition profile of the compound of formula (1) was evaluated at Proqinase (http://www.proqinase.com) by measuring residual activity values at a concentration of 0.5 and 10 μM of the test compound in singlicate in front of a panel of 300 kinases using the following protocol:

The compounds were dissolved to 1 x $10^{-03}$ M stock solutions in 100% DMSO. Subsequently, 100 μl of each stock solution were transferred into wells A3-F12 of a microtiter plate ("master plate"). Wells A1-F2 were filled with 100 μl 100% DMSO as controls. 5 x 10 μl of the master plate were aliquoted into 5 copy plates, which were stored at -20° Celsius until use. For the testing of each group of up to 8 kinases, one copy plate was used.

In the process, 90 μl $H_2O$ were added to each well of a copy plate. To minimize precipitation, the $H_2O$ was added to each well only a few minutes before the transfer of the compound solutions into the assay plates. The plate was shaken thoroughly, resulting in a "compound dilution plate" with a compound concentration of 1 x $10^{-04}$ M/10 % DMSO. This plate was used for the transfer of 5 μl compound solution into the assay plates. The final volume of the assay was 50 μl. All compounds were tested at 1 x $10^{-05}$ M in singlicate. The final DMSO concentration in the reaction cocktails was 1 % in all cases. The compound dilution plates were disposed at the end of each working day.

*Protein Kinase Assays*

A radiometric protein kinase assay ($^{33}$PanQinase® Activity Assay) was used for measuring the kinase activity of the corresponding protein kinases. All kinase assays were performed in 96-well FlashPlatesTM from Perkin Elmer (Boston, MA, USA) in a 50 μl reaction volume. The reaction cocktail was pipetted in 4 steps in the following order:

10 μl of non-radioactive ATP solution (in $H_2O$)
25 μl of assay buffer/ [γ-33P]-ATP mixture
5 μl of test sample in 10% DMSO
10 μl of enzyme/substrate mixture

The assay for all protein kinases contained 70 mM HEPES-NaOH pH 7.5, 3 mM $MgCl_2$, 3 mM $MnCl_2$, 3 μM Na-orthovanadate, 1.2 mM DTT, ATP (variable amounts, corresponding to the apparent ATP-Km of the respective kinase), [γ-$^{33}$P]-ATP (approx. 8 x $10^{05}$ cpm per well), protein kinase (variable amounts), and substrate (variable amounts).

The protein kinase reaction cocktails were incubated at 30°C for 60 minutes. The reaction was stopped with 50 μl of 2 % (v/v) $H_3PO_4$, plates were aspirated and washed two times with 200 μl 0.9 % (w/v) NaCl. All assays were performed with a BeckmanCoulter Biomek 2000/SL robotic system. Incorporation of $^{33}$Pi (counting of "cpm") was determined with a microplate scintillation counter (Microbeta, Wallac).

All protein kinase assays were performed with a BeckmanCoulter Core robotic system.

*Recombinant Kinases*

All protein kinases provided by ProQinase were expressed in Sf9 insect cells or in E.coli as recombinant GST-fusion proteins or His-tagged proteins. All kinases were produced from human cDNAs and purified by either GSH-affinity chromatography or immobilized metal. Affinity tags were removed from a number of kinases during purification. The purity of the protein kinases was examined by SDS-PAGE/Coomassie staining, the identity was checked by mass spectroscopy.

Kinases from external vendors (CAR = Carna Biosciences Inc.; INV = Life Technologies (Invitrogen Corporation); MIL = Merck-Millipore (Millipore Corporation)) were expressed, purified and quality-controlled by virtue of the vendors readings.

*Evaluation of Raw Data*

For each kinase, the median value of the cpm of six wells of column 1 of each assay plate was defined as "low control" (n=6). This value reflects unspecific binding of radioactivity to the plate in the absence of a protein kinase but in the presence of the substrate. Additionally, for each kinase the median value of the cpm of six wells of column 2 of each assay plate was taken as the "high control", i.e. full activity in the absence of any inhibitor (n=6). The difference between high and low control of each enzyme was taken as 100 % activity.

As part of the data evaluation the low control of each kinase was subtracted from the high control value as well as from their corresponding "compound values". The residual activity (in %) for each compound well was calculated by using the following formula:

$$\text{Res. Activity (\%)} = 100 \text{ X } [(\text{signal of compound} - \text{low control}) / (\text{high control} - \text{low control})]$$

A low residual activity indicates a strong inhibition of the kinase by the compound. On the contrary, a residual activity close to 100% indicates that the compound has no inhibitory effect on that particular kinase.

*Quality Controls*

As a parameter for assay quality, the Z'-factor (Zhang et al., J. Biomol. Screen. 2: 67-73, 1999) for the low and high controls of each assay plate (n = 8) was used. ProQinase's criterion for repetition of an assay plate is a Z'-factor below 0.4 (Iversen et al., J. Biomol. Screen. 3: 247-252, 2006). Z'-factors did not drop below 0.51, indicating an excellent assay quality.

*Results*

The singlicate residual activity values of the compound of formula (1) were determined. The results obtained for the kinases with residual activities ≤ 60% at 0.5 M are shown on Table 1 together with the corresponding residual activities at 10 μM.

Table 1

| *KinaseName* | *Kinase Family* | *0.5 μM* | *10 μM* |
|---|---|---|---|
| EPHA3 | TK | 9 | 36 |
| DDR2 wt | TK | 10 | 4 |
| DDR2 N456S | TK | 10 | 6 |
| EGF-R d746-750 | TK | 28 | 10 |
| EGF-R G719C | TK | 29 | 6 |
| EGF-R L861Q | TK | 36 | 8 |
| EGF-R G719S | TK | 39 | 3 |
| ABL2 | TK | 44 | 13 |

(continued)

| *KinaseName* | *Kinase Family* | *0.5 μM* | *10 μM* |
|---|---|---|---|
| EGF-R d747-752/P753S | TK | 47 | 15 |
| EGF-R wt | TK | 47 | 16 |
| EGF-R L858R | TK | 49 | 10 |
| EPHA2 | TK | 54 | 9 |
| LCK | TK | 50 | 5 |
| ERBB4 | TK | 52 | 7 |
| EPHA1 | TK | 54 | 13 |
| EPHB1 | TK | 57 | 5 |
| RAF1 Y340D/Y341D (untagged) | TKL | 57 | 25 |

**Example 2. Activity of compound of formula (1) in different tumor cell lines**

The compound of formula (1) was tested using a Thiazolyl Blue Tetrazolium Bromide (MTT) proliferation assay in a panel of tumor cell lines of different origins.

*Cell culture*

All culture materials were obtained from Biological Industries (Kibbutz Beit Haemek, Israel) or Invitrogen (Paisley, Scotland, United Kingdom). The non-tumor cell lines HF and HACAT were provided by Vall d'Hebrón Hospital and GmbH, respectively. The PC9 naïve cell line was provided by Mayumi Ono and, the H3122, HCC78 and 11-18 by Daniel Costa. The PC9-GR1, PC9-GR2, PC9-GR3, PC9-GR4, PC9-GR5 and PC9-ER cell lines were derived in house by exposing the parental PC9 cell line to increasing concentrations of erlotinib (ER) or gefitinib (GR). The rest of the cell lines in which compound (1) was tested were purchased from ATCC (H23, H1975, H2228, A549, H460, Calu-6, H1650, SK-MES-1, EBC1, SNU1, AGS, N87, PC3, BxPC3, DLD1, HT29, SW48, SCC9,FaDu, AU565, Gingival, Prostate, SU-DHL-1 and A431). All cell lines were maintained in RPMI medium supplemented with 10% FBS, 50 μg/mL penicillin-streptomycin and 2 mM L-Glutamine. All cells were grown in a humidified atmosphere with 5% CO2 at 37°C.

*Cell viability assays. Single agent*

Cell viability in response to single agent treatment was assessed by the Thiazolyl Blue Tetrazolium Bromide (MTT) (Sigma, St Louis, MO) assay. Cells from each cell line were seeded at 2000 to 10000 per well in 96-well plates. First, the concentration of drug required for 50% growth inhibition (IC50) after a 72 h treatment was assessed, and also the IC50 for compound (1) upon 72 h exposure. After treatment, cells were incubated with medium containing MTT (0.75 mg/mL in medium) for 1-2 h at 37°C. Culture medium with MTT was removed and formazan crystals reabsorbed in 100 μL DMSO (Sigma, St. Louis, MO). Cell viability was determined by measuring the absorbance at 492 nm, using a microplate reader (BioWhittaker, Walkersville, MD).

The results of activity of compound (1) in different tumor cell lines are shown in Table 2. The IC50s shown are the mean of several independent experiments. Standard errors are also listed

Table 2. SCC means squamous cell carcinoma, ADC means adenocarcinoma, GBM means glioblastoma.

| Cell Line | Tumor | Characteristics | Average IC50 (μM) | Standard Error |
|---|---|---|---|---|
| *H2286* | SCC Lung | DDR2 mutated | 0.05 | 0.01 |
| *I638F* | SCC Lung | DDR2 mutated | 0.10 | 0.02 |
| *H520* | SCC Lung | DDR2 wt | 0.35 | 0.15 |
| *A549* | Lung ADC | KRAS | 0.49 | 0.08 |
| *H1703* | SCC Lung | DDR2 wt | 0.55 | 0.15 |
| *SK-MES-1* | SCC Lung | DDR2 wt | 0.57 | 0.04 |

(continued)

| Cell Line | Tumor | Characteristics | Average IC50 (μM) | Standard Error |
|---|---|---|---|---|
| *L239R+T654M* | SCC Lung | DDR2 mutated | 0.60 | 0.19 |
| *Calu6* | Lung ADC | KRAS | 0.72 | 0.14 |
| *SW48* | Colon ADC | EGFR ex18 | 0.75 | 0.15 |
| *L239R* | SCC Lung | DDR2 mutated | 0.75 | 0.05 |
| *I638F+T654M* | SCC Lung | DDR2 mutated | 0.77 | 0.14 |
| *HCC366* | SCC Lung | DDR2 mutated | 0.83 | 0.26 |
| *PC9-GR3* | Lung ADC | EGFR ex19 | 0.88 | 0.10 |
| *HCC78* | Lung ADC | ROS1-SLC34A2 | 0.92 | 0.11 |
| *H460* | Lung ADC | KRAS | 1.00 | 0.28 |
| *435S* | Melanoma (melanocyte) | BRAF V600E | 1.00 | 0.10 |
| *DLD1* | Colon ADC | KRAS | 1.00 | 0.10 |
| *PC9-ER* | Lung ADC | EGFR ex19 | 1.18 | 0.44 |
| *H3122* | Lung ADC | ALK-EML4 (v1) | 1.34 | 0.29 |
| *A431* | SCC Melanoma | | 1.35 | 0.45 |
| *H2228* | Lung ADC | ALK-EML4 (v3) | 1.36 | 0.28 |
| *PC9-GR1* | Lung ADC | EGFR ex19 | 1.45 | 0.13 |
| *H23* | Lung ADC | KRAS | 1.57 | 0.09 |
| *WM793* | Melanoma | BRAF V600E | 1.60 | 0.37 |
| *PC9-GR5* | Lung ADC | EGFR ex19 | 1.61 | 0.16 |
| *BxPC3* | Pancreas | mut TP53 | 1.80 | 0.20 |
| *CD74-ROS1 (wt)* | Lung ADC | ROS1 translocated | 1.90 | 0.30 |
| *CD74-ROS1 (G2032)* | Lung ADC | ROS1 translocated | 2.00 | 0.20 |
| *HT29* | Colon ADC | BRAF V600E | 2.00 | 0.31 |
| *H1975* | Lung ADC | EGFR ex21 | 2.07 | 0.20 |
| *PC9-GR4* | Lung ADC | EGFR ex19 | 2.10 | 0.60 |
| *PC9-GR2* | Lung ADC | EGFR ex19 | 2.16 | 0.35 |
| *H1568* | Lung ADC | CD74-NRG1 | 2.43 | 0.18 |
| *U118MG* | GBM | ROS1-FIG | 2.60 | 0.68 |
| *H1650* | Lung ADC | EGFR ex19 | 2.68 | 0.66 |
| *PC9* | Lung ADC | EGFR ex19 | 2.85 | 0.05 |
| *UACC903* | Melanoma | BRAF V600E | 3.35 | 0.25 |
| *PC3* | Prostate ADC | DDR2 wt | 3.40 | |
| *SCC9* | SCC Head&Neck | DDR2 wt | 4.35 | 1.05 |
| *H322* | Lung BAC | CD74-NRG1 | 4.35 | 0.45 |
| *FaDu* | SCC Head&Neck | DDR2 wt | 4.85 | 2.23 |
| *H11-18* | Lung ADC | EGFR ex21 | 4.90 | 0.70 |
| *EBC-1* | SCC Lung | c-Met ampl | 5.10 | 0.15 |

It can be observed that H2286 (a cell line derived from squamous cell carcinoma of the lung carrying DDR2 mutation), H520H1703 and SK-MES-1 (cell lines derived from squamous cell carcinoma of the lung), A549 and Calu6 (carrying a KRAS mutation) and SW48 (with an EGFR mutation in exon 18, G719S) were the most sensitive cell lines.

**Example 3. Activity of combinations comprising the compound of formula (1) in different tumor cell lines**

The combinations comprising the compound of formula (1) were tested using an MTT proliferation assay in a panel of tumor cell lines of different origins. All culture materials were obtained from the sources described in Example 2. In the Chou-Talalay method, which is the standard, universally known methodology to determine drug interactions, the two drugs under study are maintained at a fixed ratio of IC50 single agent (drug 1):IC50 single agent (drug 2) while the concentration of total amount of drugs in the combination is varied and the 20% (IC20), 50% (IC50) and 80% (IC80) of growth inhibition of the combination is determined.

*Definitions*

In the activity assays on cell lines the following terms are used:

- IC50: Drug concentration at which 50% growth inhibition is achieved.
- TKI IC50: The IC50 of the tyrosine kinase inhibitor as single agent.
- Compound (1) IC50: The IC50 of compound (1) as single agent.
- CI@IC20: The combination index of the combination consisting of drug 1 and drug 2 at a weight ratio equal to IC50 of drug 1:IC50 of drug 2 wherein said combination is used at a total amount that achieves 20% of growth inhibition.
- CI@IC50: The combination index of the combination consisting of drug 1 and drug 2 at a weight ratio equal to IC50 of drug 1:IC50 of drug 2, wherein said combination is used at a total amount that achieves 50% of growth inhibition.
- CI@IC80: The combination index of the combination consisting of drug 1 and drug 2 at a weight ratio equal to IC50 of drug 1:IC50 of drug 2, wherein said combination is used at a total amount that achieves 80% of growth inhibition.
- Combination TKI IC50: The combination index at a tyrosine kinase inhibitor concentration that corresponds to the IC50 (single agent).
- The combination index (CI) concept was introduced by Chou T.C. and Talalay P.(Chou TC, Talalay P. Adv Enzyme Regul 1984;22:27-55). The derived combination index equation for two drugs is (D = dosage or concentration):

$$CI = \frac{(D)_1}{(D_x)_1} + \frac{(D)_2}{(D_x)_2}$$

where $(D_x)_1$ is the concentration of drug 1 "alone" that inhibits growth at a x% (i.e. $(D_{50})_1$ is the concentration of drug 1 "alone" that inhibits growth at a 50%), $(D_x)_2$ is the concentration of drug 2 "alone" that inhibits growth at a x%, $(D)_1$ and $(D)_2$ refer to the concentration of drug 1 and drug 2, respectively, in the combination, wherein the combination inhibits growth at the same x%. The CI Value quantitatively defines synergism (CI<1) (in particular, CI values of CI<0.7 indicates strong synergism, 0.7<CI<0.85 indicates moderate synergism, 0.85<CI<1 indicates slight synergism), additive effect (CI=1) and antagonism (CI>1). Based on the above equation, a plot of CI values at different effect levels (fa's) can be determined. Entering a series of "dose" (($D_1$), ($D_2$), (($D_x)_1$ and $(D_x)_2$) and % of growth inhibition (%x or effect (fa))" into an excel sheet for each drug alone and their combinations, the CI values at different x% (or fa levels) are obtained using the above equation. This plot is also called the Fa-CI plot or the Chou-Talalay plot.

*Cell viability assays. Combinations*

Cell viability in response to combination treatment was assessed by the Thiazolyl Blue Tetrazolium Bromide (MTT) (Sigma, St Louis, MO) assay. Cells from each cell line were seeded at 2000 to 10000 per well in 96-well plates. The effect of the combination of compound (1) with commercial drugs (crizotinib, alectinib and foretinib) was determined. Twenty-four hours after seeding, cells were exposed to compound (1), commercial drug or combinations thereof for 72 h. In the Chou-Talalay method, which is the standard, universally known methodology to determine drug interactions, the two drugs under study are maintained at a fixed ratio IC50 single agent (drug 1):IC50 single agent (drug 2). For each cell line, the 0.13; 0.25; 0.50; 0.63; 0.75; 0.88; 1; 1.5 and 3 x IC50 doses drugs were used.

In Table 3 an example of the concentrations of the two drugs under study used in a typical Chou-Talalay experiment is provided. In this case, the fixed ratio is 6.5 (IC50 drug A):25.0 (IC50 drug B). The numbers at the left of the table correspond to the concentration of mixture of the two drugs relative to the IC50.

Table 3

| | *IC50 Drug A* | *IC50 Drug B* |
|---|---|---|
| | 6.50 | 25.00 |
| *Fold 1C50* | *Drug A (μM)* | *Drug B (μM)* |
| 3.00 | 19.50 | 75.00 |
| 1.50 | 9.75 | 37.50 |
| 1.00 | 6.50 | 25.00 |
| 0.88 | 5.69 | 21.88 |
| 0.75 | 4.88 | 18.75 |
| 0.63 | 4.06 | 15.63 |
| 0.50 | 3.25 | 12.50 |
| 0.25 | 1.63 | 6.25 |
| 0.13 | 0.81 | 3.13 |

After treatment, cells were incubated with medium containing MTT (0.75 mg/mL in medium) for 1-2 h at 37°C. Culture medium with MTT was removed and formazan crystals reabsorbed in 100 μL DMSO (Sigma, St. Louis, MO). Cell viability was determined by measuring the absorbance at 492 nm, using a microplate reader (BioWhittaker, Walkersville, MD).

The nature of the interaction between commercial drug and compound (1) was determined by calculating the combination index according to the method described in Chou TC, Talalay P. Adv-Enzyme Regul 1984;22:27-55. The effect of the addition of compound (1) to the sensitivity of the cell lines for the commercial drug was also calculated, and expressed as fold-change in the IC50 for the commercial drug.

The compound of formula (1) was first tested in combination with crizotinib, ceritinib, alectinib, and foretinib in ALK translocated cell lines (H3122 and H2228) and ROS translocated cell lines (HCC78).

The results obtained in an experiment with H3122 cells are shown in Table 4, the results obtained in an experiment with H2228 cells are shown in Table 5, the results obtained in HCC78 cells are shown in Table 6.

Table 4

| *H3122 Cells* | Crizotinib | Alectinib |
|---|---|---|
| TKI IC50 μM | 0.19 | 0.05 |
| Compound (1) IC50 μM | 1.3 | 1.3 |
| CI@IC20 | 0.948 | 1.087 |
| CI@IC50 | 0.754 | 0.866 |
| CI@IC80 | 0.611 | 0.825 |
| Combination TKI IC50 μM | 0.029 | 0.015 |
| Combination Compound (1) IC50 μM | 0.500 | 0.240 |
| Fold change in the TKI IC50 | 6.552 | 3.355 |
| Type of interaction | Synergism | Weak Synergism |

In the case of the H3122 cells, a synergistic effect when combining the compound of formula (1) with crizotinib, ceritinig and alectinib was observed, particularly with crizotinib: the IC50 for this drug dropped 6.5 fold to 29 nM in presence of combining the compound of formula (1).

Table 5

| *H2228 Cells* | Crizotinib |
|---|---|
| TKI IC50 μM | 0.250 |
| Compound (1) IC50 μM | 0.809 |
| CI@IC20 | 1.315 |
| CI@IC50 | 0.910 |
| CI@IC80 | 0.685 |
| Combination TKI IC50 μM | 0.053 |
| Combination Compound (1) IC50 μM | 0.410 |
| Fold change in the TKI IC50 | 4.717 |
| Type of interaction | Synergism at CI≥IC50 |

In the case of the H2228 cell line, combining the compound of formula (1) was antagonistic with ceritinib and alectinib but showed again a remarkable synergism with crizotinib: the IC50 for this drug was 0.25 μM single agent but 53 nM when combining the compound of formula (1) was also administered.

Table 6

| *HCC78 cells* | Crizotinib | Foretinib |
|---|---|---|
| TKI IC50 μM | 7.160 | 0.37 |
| Compound (1) IC50 μM | 0.8 | 0.8 |
| CI@IC20 | 0.631 | 0.731 |
| CI@IC50 | 0.739 | 0.787 |
| CI@IC80 | 0.871 | 0.865 |
| Combination TKI IC50 μM | 0.249 | 0.130 |
| Combination Compound (1) IC50 μM | 0.430 | 0.210 |
| Fold change in the TKI IC50 | 28.755 | 2.846 |
| Type of interaction | Synergism | Synergism |

In the case of the ROS translocated cell line (HCC78), a strong synergistic effect of the compound of formula (1) with crizotinib was observed again. The IC50 of the drug (crizotinib) was 7.2 μM single agent but 0.25 μM when the compound of formula (1) was present, representing an almost 30-fold decrease in the IC50.

## Claims

**1.** Compound of formula (1)

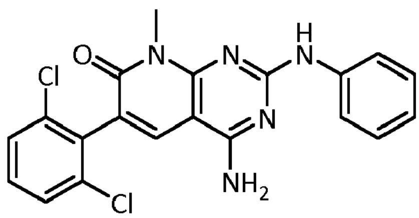

(1)

or a pharmaceutically acceptable salt or solvate thereof, for use in the treatment and/or prevention of solid cancers.

2. Compound of formula (1) or a pharmaceutically acceptable salt or solvate thereof for use according to claim 1, wherein the solid cancer is selected from the group consisting of carcinoma, glioblastoma multiforme, astrocytoma, oligodendroglioma and ependymoma.

3. Compound of formula (1) or a pharmaceutically acceptable salt or solvate thereof for use according to claim 2, wherein the solid cancer is a carcinoma selected from the group consisting of lung cancer, colorectal cancer, pancreatic cancer, larynx cancer, tongue cancer, prostate cancer, breast cancer, ovarian cancer, liver cancer, head and neck cancer, esophageal cancer, renal cancer, endometrial cancer, gall bladder cancer, bladder cancer and gastric cancer.

4. Compound of formula (1) or a pharmaceutically acceptable salt or solvate thereof for use according to claim 3, wherein the solid cancer is lung cancer.

5. Compound of formula (1) or a pharmaceutically acceptable salt or solvate thereof for use according to claim 4, wherein the lung cancer is non-small cell lung cancer (NSCLC).

6. Compound of formula (1) or a pharmaceutically acceptable salt or solvate thereof for use according to claim 5, wherein the non-small cell lung cancer is selected form the group consisting of squamous cell carcinoma, adenocarcinoma and large cell carcinoma.

7. Compound of formula (1) or a pharmaceutically acceptable salt or solvate thereof for use according to claim 6, wherein the non-small cell lung cancer is squamous cell carcinoma.

8. Compound of formula (1) or a pharmaceutically acceptable salt or solvate thereof for use according to claim 4 wherein the lung cancer is SCLC.

9. Compound of formula (1) or a pharmaceutically acceptable salt or solvate thereof for use according to any one of claims 1 to 8, wherein the solid cancer is selected from the group consisting of a solid cancer having ALK translocations, ROS-1 translocations, KRAS mutations, RET translocations, DDR2 mutations, EGFR mutations, FGFR1 amplification, SOX2 amplification and/or PIK3CA mutations, and a solid cancer having over expression of DDR2, EGFR and/or EPHA3.

10. Combination comprising a compound of formula (1) or a pharmaceutically acceptable salt or solvate thereof and a chemotherapeutic drug.

11. Combination according to claim 10, wherein the chemotherapeutic drug is selected from the group consisting of EGFR TKIs selected from gefitinib, erlotinib, icotinib hydrochloride, foretinib, rociletinib, canetinib, ABT-414, ASP8273, EGF-816, PF-06747775, AZD3759, HM61713, LY-3164530, NRC2694, and TAS 120; ALK TKIs selected from crizotinib, ceritinib, alectinib, entrectinib, gliteritinib fumarte, PF-06463922, CEP-37440, EBI-215, AZD3463, CEP28122, CM-118, TSR-011, X-396, DLX-521, and AP26113; anti-EGFR monoclonal antibodies selected from cetuximab, panitumumab, nimotuzumab, necitumumab, ABT-806, duligotuzumab, futuximab, imgatuzumab, and GC-1118A; anti-VEGF monoclonal antibodies selected from bevacizumab, ramucirumab, icrucumab, tanibirumab, vanucizumab, LY3022856, MP0250, OMP305B83, sevacizumab, and VGX100; aflibercept; multi-tyrosine kinase inhibitors selected from afatinib, imatinib, dacomitinib, dasatinib, ponatinib, KD-019, bosutinib, lapatinib ditosylate, AZD9291, neratinib, poziotinib, S-222611, suramin hexasodium, AL-6802, BGB-102, PB357, Pyrotinib, sunitinib, sorafenib tosylate, pazopanib, regorafenib, apatinib, axitinib, carbozantinib, lenvatinib, nintedanib, vandetanib, tivozanib, anlotinib, midostaurin, muparfostat, BMS-690514, ENMD-2076, golvatinib, lucitanib, motesanib, necuparinib, RAF265, famitinib, telatinib, X82, ALNVSP, altiratinib, ABT348, MGCD516, OB318, ODM203, PG545, nilotinib, nintedanib, masitinib, AL3818, flumatinib, crenolanib besylate, HHGV678, LY-3012207, CS2164, ilorasertib, radotinib, bafetinib, NRCAN-019, ABL001, metatinib tromethamine, rebastinib tosylate, VX-15, and Herceptin; immuno inhibitors selected from alemtuzumab, gemtuzumab, rituximab, tositumomab, zevalin, and epratuzumab; oregovomab; olaparib; etoposide; topotecan; teniposide; mitoxantrone; cisplatin; mechlorethamine; cyclosphosphamide; ifosfamide; melphalan; procarbazine; bleomycin; doxorubicin; daunorubicin; idarubicin; mitomycin; vincristine; vinblastine; cytarabine; hydroxyurea; 5-fluorouracil; thioguanine; irinotecan; fludarabine; pentostatin; cladribine; lenalidomide; paclitaxel; abraxane; and docetaxel.

12. Combination according to claim 11, wherein the chemotherapeutic drug is crizotinib.

**13.** Combination as defined in any one of claims 10 to 12 for use in medicine.

**14.** Combination as defined in any one of claims 10 to 12 for use in the treatment and/or prevention of solid cancers as defined in any one of claims 1 to 9.

**15.** Pharmaceutical composition comprising a combination as defined in any one of claims 10 to 12 and a pharmaceutically acceptable excipient.

Europäisches Patentamt
European Patent Office
Office européen des brevets

# EUROPEAN SEARCH REPORT

Application Number
EP 15 38 2375

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | EP 2 813 504 A1 (INST QUIMIC DE SARRIÁ CETS FUNDACIÓ PRIVADA [ES]; HOSPITAL CLÍNIC DE B) 17 December 2014 (2014-12-17)<br>* claims; 0081 * | 10,13,15<br><br>10-13,15 | INV.<br>A61K31/519<br>ADD.<br>A61P35/00 |
| Y | DIDIER DECAUDIN ET AL: "In vivo efficacy of STI571 in xenografted human small cell lung cancer alone or combined with chemotherapy",<br>INTERNATIONAL JOURNAL OF CANCER,<br>vol. 113, no. 5,<br>20 February 2005 (2005-02-20), pages 849-856, XP055215135,<br>ISSN: 0020-7136, DOI: 10.1002/ijc.20652<br>* abstract * | 10-13,15 | |
| Y | EMILEIGH K. GREUBER ET AL: "Role of ABL family kinases in cancer: from leukaemia to solid tumours",<br>NATURE REVIEWS CANCER,<br>vol. 13, no. 8, 11 July 2013 (2013-07-11), pages 559-571, XP055215234,<br>ISSN: 1474-175X, DOI: 10.1038/nrc3563<br>* title, abstract, p. 9, last * | 10-13,15 | TECHNICAL FIELDS SEARCHED (IPC)<br>A61K |
| Y | C. GAMBACORTI PASSERINI ET AL: "Crizotinib in Advanced, Chemoresistant Anaplastic Lymphoma Kinase-Positive Lymphoma Patients",<br>JNCI JOURNAL OF THE NATIONAL CANCER INSTITUTE,<br>vol. 106, no. 2,<br>3 February 2014 (2014-02-03), pages djt378-djt378, XP055215420,<br>ISSN: 0027-8874, DOI: 10.1093/jnci/djt378<br>* title, abstract * | 10-13,15 | |

The present search report has been drawn up for all claims

1

| Place of search | Date of completion of the search | Examiner |
|---|---|---|
| Munich | 22 September 2015 | Dahse, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 38 2375

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2015

| Patent document cited in search report | Publication date | Patent family member(s) | Publication date |
|---|---|---|---|
| EP 2813504 A1 | 17-12-2014 | EP 2813504 A1 | 17-12-2014 |
| | | WO 2014198960 A1 | 18-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *GLOBALCAN2012, WHO, Globalcan2012: Estimated cancer incidence, mortality and prevalence worldwide,* 2012
- **R. ROSELL et al.** *Lancet Oncol,* 2012, vol. 13, 239-246
- **E.L. KWAK et al.** *N Engl J Med,* 2010, vol. 363, 1693-1703
- **T. TAKAHASHI et al.** *Ann Surg Oncol,* 2010, vol. 17, 889-897
- **E.L. KWAK et al.** *NEngl J Med,* 2010, vol. 363, 1693-1703
- **K. RIKOVA et al.** *Cell,* 2007, vol. 131, 1190-1203
- **S.H. OU et al.** *Expert Rev Anticancer Ther,* 2012, vol. 12, 447-456
- **TAKEUCHI et al.** *Nat Med,* 2012, vol. 18, 378-381
- **K. BERGETHON et al.** *J Clin Oncol,* 2012, vol. 30, 863-870
- **R. PUIG DE LA BELLACASA et al.** *Eur. J. Med. Chem.,* 2014, vol. 86, 664-675
- **R. PUIG DE LA BELLACASA et al.** *Eur. J. Med. Chem,* 2014, vol. 86, 664-675
- **PUIG DE LA BELLACASA et al.** *Eur. J. Med. Chem,* 2014, vol. 86, 664-675
- **MOLINA-VILA et al.** *J Thorac Oncol.,* 2008, vol. 3 (11), 1224-35
- **FERRI et al.** *Am J Pathol.,* 2004, vol. 164 (5), 1575-85
- **ZHANG et al.** *J. Biomol. Screen,* 1999, vol. 2, 67-73
- **IVERSEN et al.** *J. Biomol. Screen.,* 2006, vol. 3, 247-252
- **CHOU TC ; TALALAY P.** *Adv Enzyme Regul,* 1984, vol. 22, 27-55
- **CHOU TC ; TALALAY P.** *Adv-Enzyme Regul,* 1984, vol. 22, 27-55